# EUROPEAN PATENT SPECIFICATION

(11) **EP 1 315 702 B1**
(45) Date of publication and mention of the grant of the patent: **10.11.2004**
(21) Application number: 01966618.9
(22) Date of filing: 07.09.2001
(51) Int. Cl.: C07D 215/22

(54) **PROCESS TO PRODUCE HALO-4-PHENOXYQUINOLINES**
VERFAHREN ZUR HERSTELLUNG VON HALO-4-PHENOXY-CHINOLINEN
PROCEDE DE PRODUCTION DE HALO-4-PHENOXYQUINOLINES

(30) Priority: 08.09.2000 US 231719 P
(43) Date of publication of application: 04.06.2003
(73) Proprietor: Dow AgroSciences LLC, Indianapolis, Indiana 46268 (US)
(72) Inventor: KRUMEL, Karl, Leopold, Midland, MI 48640 (US); REED, Ruth, Cezar, Saginaw, MI 48604 (US); OLMSTEAD, Thomas, A., San Diego, CA 92129 (US); ROTH, Gary, Alan, Midland, MI 48440 (US); KING, Ian, Robert, King's Lynn, Norfolk PE30 3NQ (GB); BRATTESANI, Donald, Neil, Oakland, CA 94610 (US); CAMPBELL, Kent, Douglas, Concord, CA 94521 (US); DAVIES, John, Midland, MI 48642 (US)
(74) Representative: Dey, Michael, Dr.
(86) International application number: PCT/US2001/027689
(87) International publication number: WO 2002/020490

(56) References cited:
- EP-A- 0 569 021
- WO-A-98/33774
- WO-A-98/51146
- DE, D. ET AL.: "Structure-Activity Relationships for Antiplasmodial Activity among 7-Substituted 4-Aminoquinolines" JOURNAL OF MEDICINAL CHEMISTRY, vol. 41, 1998, pages 4918-4926, XP002192683
- PRICE C C ET AL: "THE SYNTHESIS OF 4-HYDROXYQUINOLINES I. THROUGH ETHOXYMETHYLENEMALONIC ESTER" JOURNAL OF THE AMERICAN CHEMICAL SOCIETY, AMERICAN CHEMICAL SOCIETY, WASHINGTON, DC, US, vol. 68, 1 July 1946 (1946-07-01), pages 1204-1208, XP002064549 ISSN: 0002-7863

## Description

### Priority

This application claims priority from U.S. provisional application serial number 60/231,719 which was filed on September 8, 2000.

### Field of the Invention

This invention is related to the field of processes to produce halo-4-phenoxyquinolines.

### Background of the Invention

Our history is riddled with outbreaks of fungal diseases that have caused widespread human suffering. One need look no further than the Irish potato famine of the 1850's, where an estimated 1,000,000 people died, to see the effects of a fungal disease.

Fungicides are compounds, of natural or synthetic origin, which act to protect plants against damage caused by fungi. Current methods of agriculture rely heavily on the use of fungicides. In fact, some crops cannot be grown usefully without the use of fungicides. Using fungicides allows a grower to increase the yield of the crop and consequently, increase the value of the crop. In most situations, the increase in value of the crop is worth at least three times the cost of the use of the fungicide.

However, research is being conducted to lower the cost of producing fungicides, thereby lowering the costs associated with using fungicides. In light of the above, the inventors provide this invention.

### Summary of the Invention

It is an object of this invention to provide a process to produce halo-4-phenoxyquinolines. In accordance with this invention a process is provided, said process comprising:
(1) reacting a compound of formula one with a compound of formula two to produce a compound of formula three;
(2) reacting said compound of formula three to produce a compound of formula four;
(3) reacting said compound of formula four with a compound of formula five to produce a compound of formula six;
(4) reacting said compound of formula six with a compound of formula seven to produce a compound of formula eight; and
(5) reacting said compound of formula eight with a compound of formula nine to produce a compound of formula ten.

### Detailed Description of the Invention

The first step in this process is reacting a compound of formula one with a compound of formula two to produce a compound of formula three, as shown in the reaction scheme.

In the compound of formula one (also referred to herein as "Compound One" or "Aniline Compound") R¹ and R³ are independently halo and R² and R⁴ are H; or R³ is halo, R¹ is halo or H, and R² and R⁴ are H; or R⁴ is halo and R¹ to R³ are H. Currently, it is preferred when 3,5-dichloroaniline (also referred to herein as "DCA") is used as Compound One.

In the compound of formula two (also referred to herein as "Compound Two" or "Alkoxymethylene Malonate Dialkyl Ester Compound") R⁵ is a (C₁-C₄) alkyl and R⁶ and R⁷ are independently a (C₁-C₄) alkyl. Currently, it is preferred when diethyl ethoxymethylenemalonate (also referred to herein as "EMME") is used as Compound Two.

The compound of formula three is also referred to herein as "Compound Three" or "Aniline-Alkoxymethylene Malonate Dialkyl Ester-Product Compound". The R groups are the same as defined in Compound One and Compound Two.

The reaction is conducted by subjecting a reaction mixture comprising Compound One and Compound Two to a temperature from about 50 to about 210°C, preferably from about 160 to about 210°C, and a pressure from about 5 psia to about 50 psia, for a time period sufficient to drive the reaction to completion, usually about 0.5-3 hours. Currently, it is preferred when a slight molar excess of the Compound Two to Compound One is used.

This step of the process can be conducted in the presence of a solvent, however, a solvent is not required. Any solvent that does not react with Compound One or Compound Two under the reaction conditions of this step is useful. Preferably this solvent has a boiling point above the reaction temperature when subjected to the reaction conditions. Solvents such as, for example, sulfolane, tetraglyme, polyethers, long-chain (C₁₀₋₄₀) alkylaromatics, C₁₋₆ alkylated naphthalene, naphthalene, diesel or fuel oil can be suitable. Currently, it is preferred if a hydrocarbyl aromatic compound, preferably a (C₁-C₃₀) alkyl substituted hydrocarbyl aromatic compound, is used. Currently, it is more preferred to use dodecylbenzene, however, in practical terms, it is probably less expensive to use a composition that contains mixtures of (C₁-C₃₀)alkyl-substituted aryl compounds.

The second step in this process is reacting the compound of formula three to produce a compound of formula four, as shown in the reaction scheme.

In the compound of formula four (also referred to herein as "Compound Four" or "Alkoxycarbonyl Quinoline Compound") R⁸ is a (C₁-C₄) alkyl.

The reaction is conducted by subjecting a reaction mixture comprising Compound Three to a temperature from about 200-270°C, preferably from about 220-250°C, and a pressure from about 5 psia to about 50 psia, for a time period sufficient to drive the reaction to completion, usually about 2-20 hours.

This step of the process is preferably conducted in the presence of a solvent. The solvents discussed above in step one can be used.

Optionally, this step can include recovering said Compound Four before proceeding to step three of the process. This recovery can be accomplished by any means which isolates Compound Four such as, for example, as discussed in U.S. patent 5,973,153 (hereby incorporated by reference). If Compound Four is isolated it can optionally be converted to an acid by exchanging the R⁸ for hydrogen (-H).

The third step in this process is reacting the compound of formula four with a compound of formula five to produce a compound of formula six, as shown in the reaction scheme.

The compound of formula five (also referred to herein as "Compound Five" or "Hydroxy Compound") is any compound that contains a hydroxy group (-OH) and that interacts with the -C(=O)-O-R⁸ group on Compound Four to produce a compound of formula six (also referred to herein as "Compound Six" or "Hydroxy Quinoline Compound"). Currently, H₂O is the preferred compound to use as Compound Five.

The reaction is conducted by subjecting a reaction mixture comprising Compound Four and Compound Five to a temperature from about 180-270°C, preferably from about 200-230°C, and a pressure from about 5 psia to about 50 psia, for a time period sufficient to drive the reaction to completion, usually about 2-20 hours.

This step of the process is preferably conducted in the presence of a solvent. The solvents discussed above in step one can be used. Currently, it is preferred if a polar hydrocarbylhetero compound, preferably a (C₁-C₁₀) polar hydrocarbylhetero compound, is used. Currently, it is more preferred to use sulfolane. It is preferred to use a weight ratio of 5:1 to 20: solvent:Compound Four.

If desired it is possible to isolate Compound Six before reacting it with Compound Seven in a solvent. Additionally, if desired it is possible to dry Compound Six before reacting it with Compound Seven in a solvent.

The fourth step in the this process is reacting the compound of formula six with a compound of formula seven to produce a compound of formula eight, as shown in the reaction scheme.

In the compound of formula seven (also referred to herein as "Compound Seven" or "Halo Compound") is any compound that contains a halo group (-F, Cl, Br, or I) and that interacts with the -OH group on Compound Six to produce a compound of formula Eight (also referred to herein as "Compound Eight" or "Halo Quinoline Compound"). Currently, SOCl₂ (aka thionyl chloride) is the preferred compound to use as Compound Seven.

The reaction is conducted by subjecting a reaction mixture comprising Compound Six and Compound Seven to a temperature from about 50-100°C, preferably from about 60-90°C, and a pressure from about 5 psia to about 50 psia, for a time period sufficient to drive the reaction to completion, usually about 0.5-5 hours.

This step of the process is preferably conducted in the presence of a solvent The solvents discussed above in step one can be used. Currently, it is preferred when a polar hydrocarbylhetero compound is used. It is also preferred to use a catalyst to increase to rate of reaction. Currently, N,N-dimethylformamide and N,N-diethylformamide are preferred catalysts.

Currently, it is preferred to remove any of Compound Seven or other unwanted side products before proceeding to step five of the process.

The fifth step in this process is reacting the compound of formula eight with a compound of formula nine to produce a compound of formula ten, as shown in the reaction scheme.

In the compound of formula nine (also referred to herein as "Compound Nine" or "Aryloxy Compound") R⁹ to R¹³ are independently H, CN, NO₂, OH, halo, (C₁-C₄)alkyl, (C₂-C₄)alkanoyl, halo(C₁-C₇)alkyl, hydroxy(C₁-C₇)alkyl, (C₁-C₇)alkoxy, halo(C₁-C₇)alkoxy, (C₁-C₇)alkylthio, halo(C₁-C₇)alkylthio, phenyl, substituted phenyl, phenoxy, substituted phenoxy, phenylthio, substituted phenylthio, phenyl(C₁-C₄)alkyl, substituted phenyl(C₁-C₄)alkyl, benzoyl, SiR²⁰R²¹R²², or OSiR²⁰R²¹R²² where R²⁰ ,R²¹, and R²² are H, a (C₁-C₆)alkyl group, phenyl, or substituted phenyl, provided that at least one of R²⁰, R²¹, and R²² is other than H, or R¹¹ and R¹² or R¹² and R¹³ combine to form a carbocyclic ring, and provided that unless all of R⁹ to R¹³ are H or F, then at least two of R⁹ to R¹³ are H. Wherein the foregoing definitions, the term substituted phenyl refers to phenyl substituted with up to three groups selected from halo, (C₁-C₁₀)alkyl, halo(C₁-C₇)alkyl, hydroxy(C₁-C₇)alkyl, (C₁-C₇)alkoxy, halo(C₁-C₇)alkoxy, phenoxy, phenyl, NO₂, OH, CN, (C₁-C₄)alkanoyloxy, or benzyloxy. The term alkyl refers to linear, branched, or cyclic alkyl. The term halo refers to fluoro, chloro, bromo, or iodo. The term substituted phenoxy refers to a phenoxy group substituted with up to three groups selected from halo, (C₁-C₁₀)alkyl, halo(C₁-C₇)alkyl, hydroxy-(C₁-C₇)alkyl, (C₁-C₇)alkoxy, halo(C₁-C₇)alkoxy, phenoxy, phenyl, NO₂, OH, CN, (C₁-C₄)alkanoyloxy, or benzyloxy. The term substituted phenylthio refers to a phenylthio group substituted with up to three groups selected from halo, (C₁-C₁₀)alkyl, halo(C₁-C₇)alkyl, hydroxy(C₁-C₇)alkyl, (C₁-C₇)alkoxy, halo(C₁-C₇)alkoxy, phenoxy, phenyl, NO₂, OH, CN, (C₁-C₄)alkanoyloxy, or benzyloxy. Currently, it is preferred to use parafluorophenol as Compound Nine.

The reaction is conducted by subjecting a reaction mixture comprising Compound Eight and Compound Nine to a temperature from about 40-70°C, preferably from about 55-65°C, and a pressure from about 0.5 psia to about 50 psia, for a time period sufficient to drive the reaction to completion, usually about 0.5-20 hours.

This step of the process is preferably conducted in the presence of a solvent. The solvents discussed above in step one can be used. Currently, it is preferred when a polar hydrocarbylhetero compound is used.

Currently, it is preferred to use a base during the reaction. Any base that promotes this coupling reaction can be used. Currently it is preferred when the base contains a -OH group. Suitable bases are triethylamine, sodium carbonate, potassium carbonate, sodium hydroxide, and potassium hydroxide. Currently, potassium hydroxide is preferred.

Optionally, step five can include recovering said compound of formula ten. This recovery can be accomplished by any means that isolates Compound Ten such as, for example, as discussed in U.S. Patent 5,973,153.

Currently, it is preferred to use dodecylbenzene as a solvent in steps one and two, and sulfolane as a solvent in steps 3-5. Additionally, it is preferred to isolate Compound Four before proceeding to step three.

### Examples

These examples are provided to further illustrate the invention to one skilled in the art.

### Example One: Preparation of fluoro-4-phenoxy-5,7-dichloroquinoline.

The compounds 3,5-dichloroaniline (DCA, 162.7g, 1.0 mole) and diethyl ethoxymethylenemalonate (EMME, 221.1g, 1.02 mole) were mixed into 1.135 kg of recycle dodecylbenzene and 83.3 g of fresh dodecylbenzene. The reaction was carried out in a stainless steel 3-liter, 3-neck round bottom flask equipped with a mechanical agitator and Dean Stark distillation apparatus. The reaction mixture was agitated and nitrogen purged below the liquid level at a rate of 200cc/min. The reaction mixture was heated to 180°C over a 60 minute period, then heated to 200°C for 30 minutes, collecting about one equivalent of ethanol overhead in a Dean Stark apparatus. The mixture was then cooled to room temperature and left overnight under a nitrogen purge.

The reaction mixture was then heated to 240° C for about 7 hours while purging nitrogen below the liquid level at 180 cc/min and agitating at about 450 RPM. Approximately one equivalent of ethanol was collected overhead during this period. The mixture was cooled to 130°C and filtered hot using a Buchner funnel with Whatman number 1 paper. The cake was washed with 150ml of fresh dodecylbenzene and the total filtrate (1066.3g) removed for recycle. The cake was then washed with two 377g (500ml) of recycle ethanol followed by one washed with 377g of distilled ethanol. The cake was then washed with 500ml of hot (50° C) water. This yielded a 439.7 g water wet-cake of Compound Four.

The wet cake, 431.4g (after sample removal), was then added to 1.8 kg (about 1.43 Liter) of recycle sulfolane in a 5-Liter glass reactor. The residual water (from the wet cake) was distilled at atmospheric pressure from the solution at 90° to 215° C, recovering about 130.9g of water (some water lost with the vent gases). Care was taken not to super-heat the mixture and nitrogen was purged through the reactor at about 200cc/min. After reaching a temperature of 200°C the nitrogen purge was removed and the mixture slowly heated to 220°C. Water was then added at a rate of 6g/hour over a five hour period, collecting 56g of ethanol/water in a Dean Stark trap, while maintaining a temperature of 219°C. Heating continued at 220°C for an additional 2 hours to insure complete reaction. The reaction mixture was then cooled overnight. The reaction mixture was then heated to 120°C and the residual water removed at reduced pressure (25mm Hg) for one hour. The reaction mixture was then treated with 3.71g (0.05 mole) of dimethylformamide and 155.7g (1.31 mole) of thionyl chloride at a temperature of 75°C for two hours. The excess thionyl chloride was then removed at reduced pressure (25mm Hg) at 90°C for 2 hours recovering 25.7g of thionyl chloride and sulfur dioxide in the initial trap. The contents were then kept under a nitrogen pad at room temperature over a 3-day weekend (trichloroquinoline compound mixture).

A 5 liter stainless steel reactor was charged with 102.1 g (0.91 mole) of parafluorophenol and 300.7g (2.41 mole) of 45% potassium hydroxide and mixed at room temperature. This trichloroquinoline solution (in the glass reactor) was mixed and heated to 50°C to increase the mobility of the slurry and to break-up any solids that formed. The trichloroquinoline mixture was then added to the solution in the 5-liter stainless steel reactor, noting an exotherm going from 21°C to 51°C. The reactor was slurried with 81.3g of fresh sulfolane and the mixture used to flush solids that were stuck to the funnel used to add the slurry. The reaction mixture was then heated to 66°C for four hours at an agitation rate of 600 RPM. After the 4 hours of heating at 66°C, a water addition pump was set-up and water added to the reaction mixture using an accelerated 4-tier rate. For 40 minutes water was added at a rate of 5ml/min. Then for the next 20 minutes the rate was increased to 10ml/min. The rate was doubled to 20ml/min for the next 10 minutes and finally 40ml/min (15 minutes) until 1200ml of water had been added to the reactor. The mixture was stirred and cooled to 50°C prior to emptying the reactor in a 4-liter beaker. The exit valve of the reactor was flushed with 227ml of fresh water, which was added to the quenched reaction mixture. The reaction mixture was then filtered and washed with 500ml of hot (50°C) water recovering 3665.3g of filtrate solution for sulfolane recovery. The wet cake was then washed a second time with 500ml of hot water recovering 342.8g of water-wet cake which contained Compound Ten.

### Example Two: Preparation of fluoro-4-phenoxy-5,7-dichloroquinoline.

This example illustrates steps 3-5 of the process. 7018 kg of wet (∼3 percent by weight water) sulfolane was loaded into a reactor followed by loading 906 kg of 3,5-dichloro-3-carboxy-4-hydroxyquinoline to form a wet slurry. The slurry was heated to 220°C at atmospheric pressure to remove the majority of the water and held at that temperature for 5 hours to produce 3,5-dichloro-4-hydroxyquinoline. This mixture was cooled to 105°C and full vacuum was applied for 3 hours to remove water. The mixture was then transferred to another reactor using an additional 750 kg of sulfolane to flush the transfer lines, and further cooled to 70°C then 522 kg of thionyl chloride and 13.4 kg N,N-dimethylformamide were added and the 3,5-dichloro-4-hydroxyquinoline was reacted at 75°C for 2 hours to produce 4,5,7-trichloroquinoline. The mixture was then heated to 95°C with full vacuum for 3 hours to remove SO₂ and SOCl₂, then cooled to 60°C prior to transfer. The next reactor was preloaded with 1077 kg of 45% KOH aqueous solution and 373 kg parafluorophenol before receiving the 4,5,7-trichloroquinoline mixture and receiving an additional 600 kg of sulfolane to flush the lines. This mixture was then reacted at 60°C for 12 hours without water removal to make fluoro-4-phenoxy-5,7-dichloroquinoline. 5760 kg of water was then added over 4 hours and then cooled to 40°C to form the product crystals. The mixture was then fed to two centrifuges to recover the product. The solids were washed with water.

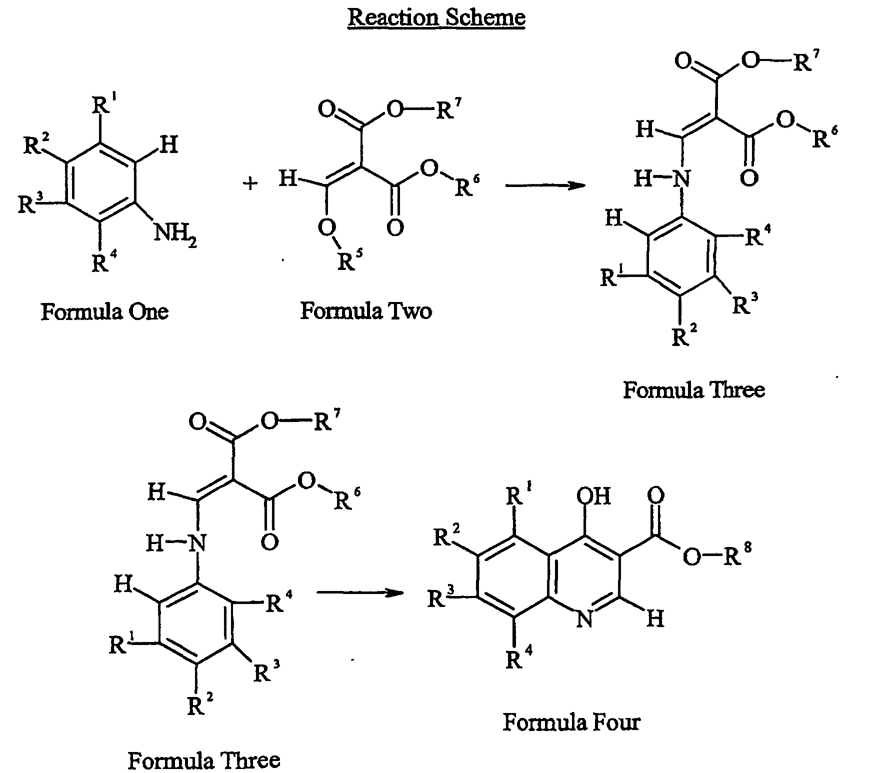

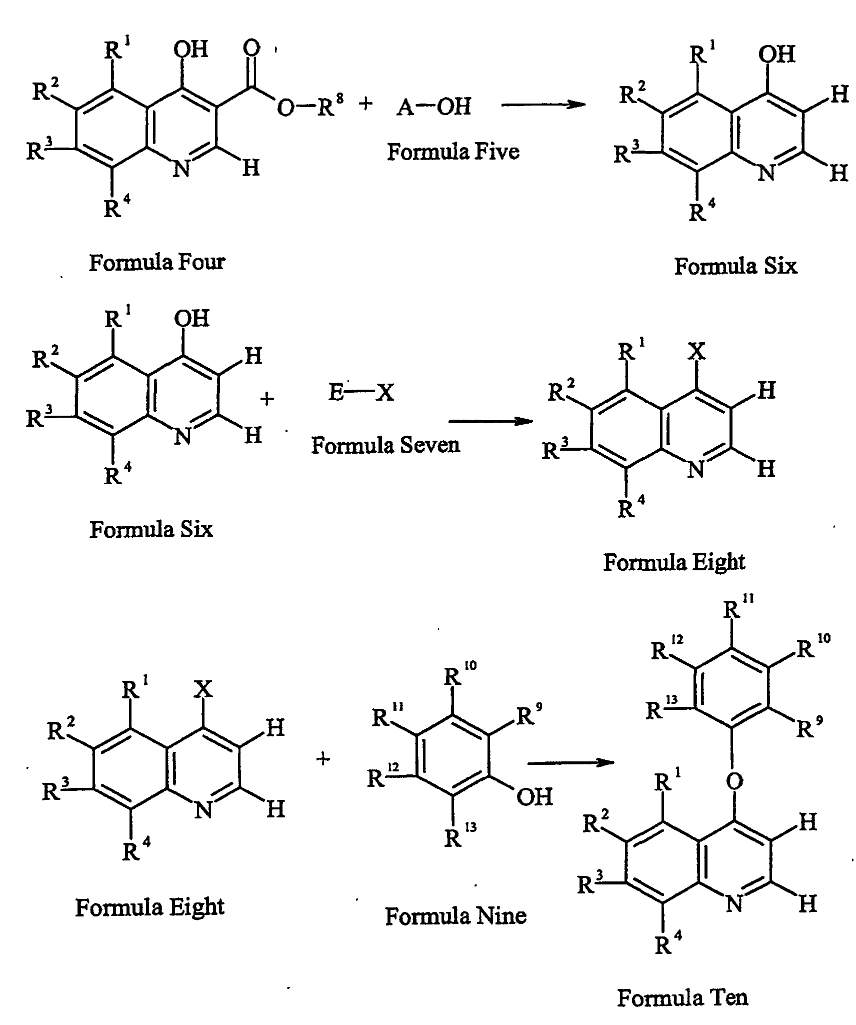

## Claims

1. A process comprising:
(1) reacting a compound of formula one with a compound of formula two to produce a compound of formula three 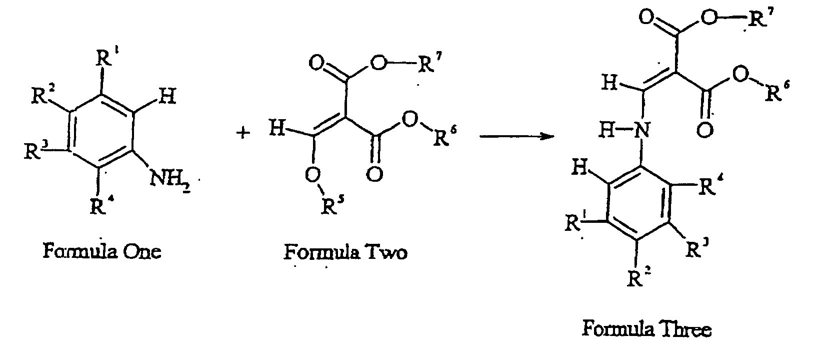 wherein R¹ and R³ are independently halo and R² and R⁴ are H; or R³ is halo, R¹ is halo or H, and R² and R⁴ are H; or R⁴ is halo and R¹ to R³ are H; R⁵ is a (C₁-C₄) alkyl and R⁶ and R⁷ are independently a (C₁-C₄) alkyl;
(2) reacting said compound of formula three to produce a compound of formula four 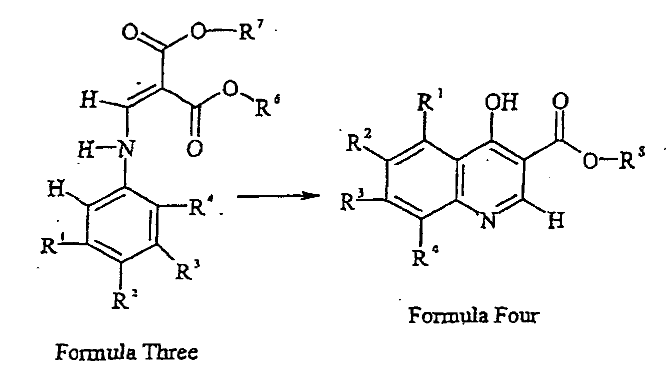 wherein R⁸ is a (C₁-C₄) alkyl;
(3) reacting said compound of formula four with a compound of formula five to produce a compound of formula six 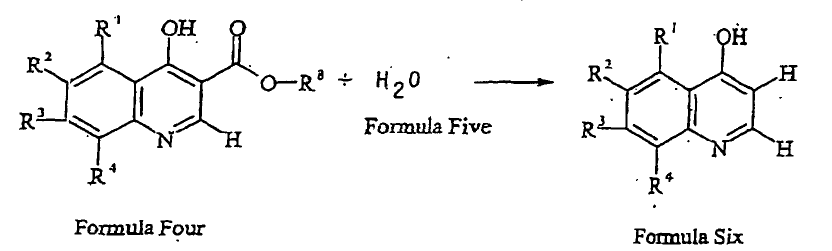 wherein H₂O interacts with the -C(=O)-O-R⁸ group on Compound Four to produce a compound of formula six;
(4) reacting said compound of formula six with a compound of formula seven to produce a compound of formula eight 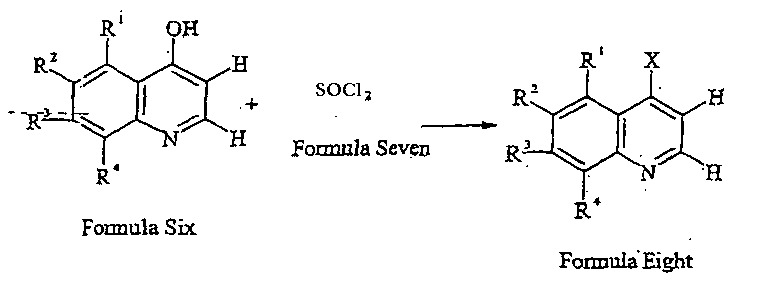 wherein SOCl₂ interacts with the -OH group on Compound Six to produce a compound of Formula Eight; and
(5) reacting said compound of formula eight with a compound of formula nine to produce a compound of formula ten 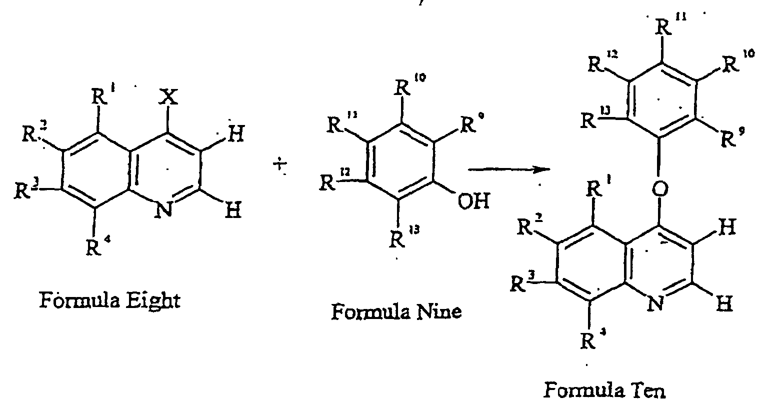 wherein R⁹ to R¹³ are independently H, CN, NO₂, OH, halo, (C₁-C₄)alkyl, (C₂-C₄)alkanoyl, halo(C₁-C₇)alkyl, hydroxy(C₁-C₇)alkyl, (C₁-C₇)alkoxy, halo(C₁-C₇)alkoxy, (C₁-C₇)alkylthio, halo(C₁-C₇)alkylthio, phenyl, substituted phenyl, phenoxy, substituted phenoxy, phenylthio, substituted phenylthio, phenyl(C₁-C₄)alkyl, substituted phenyl(C₁-C₄)alkyl, benzoyl, SiR²⁰R²¹R²², or OSiR²⁰R²¹R²² where R²⁰ ,R²¹, and R²² are H, a (C₁-C₆)alky group, phenyl, or substituted phenyl, provided that at least one of R²⁰, R²¹, and R²² is other than H, or R¹¹ and R¹² or R¹² and R¹³ combine to form a carbocyclic ring, and provided that unless all of R⁹ to R¹³ are H or F, then at least two of R⁹ to R¹³ are H;.

2. A process according to claim 1 wherein at least one of steps (1) - (5) is conducted in a solvent.

3. A process according to claim 2 wherein said solvent is selected from the group consisting of sulfolane, tetraglyme, polyethers, long-chain (C₁₀₋₄₀) alkylaromatics, C₁₋₆ alkylated naphthalene, naphthalene, diesel, fuel oil, and mixtures thereof.

4. A process according to claim 3 wherein said compound of formula one is 3,5-dichloroaniline and said compound of formula two is diethyl ethoxymethylenemalonate.

5. A process according to claim 4 wherein dodecylbenzene is used as said solvent in steps (1) and (2) and sulfolane is used as said solvent in steps (3)-(5).

6. A process according to claim 1, wherein step (3) is conducted at a temperature of from 180-270°C and at a pressure of from 5 psia to 50 psia.

## Patentansprüche

1. Verfahren, umfassend:
(1) Umsetzung einer Verbindung der Formel eins mit einer Verbindung der Formel zwei, um eine Verbindung der Formel drei darzustellen 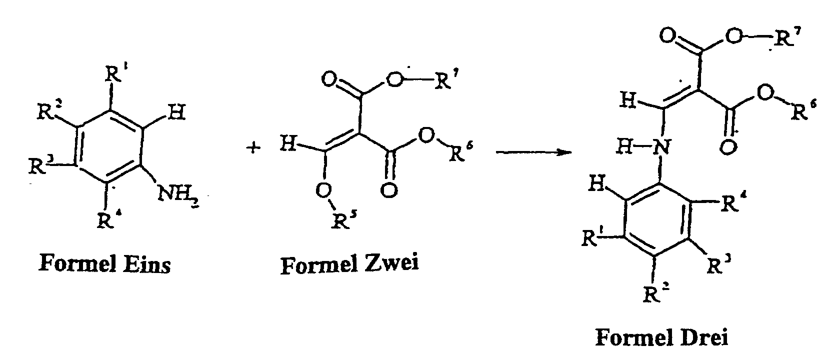 worin R¹ und R³ unabhängig Halogen sind und R² und R⁴ Wasserstoff sind; oder R³ ist Halogen, R¹ ist Halogen oder Wasserstoff, und R² und R⁴ sind Wasserstoff; oder R⁴ ist Halogen und R¹ bis R³ sind Wasserstoff; R⁵ ist ein (C₁-C₄)-Alkyl und R⁶ und R⁷ sind unabhängig ein (C₁-C₄)-Alkyl;
(2) Umsetzung der Verbindung der Formel drei, um eine Verbindung der Formel vier darzustellen 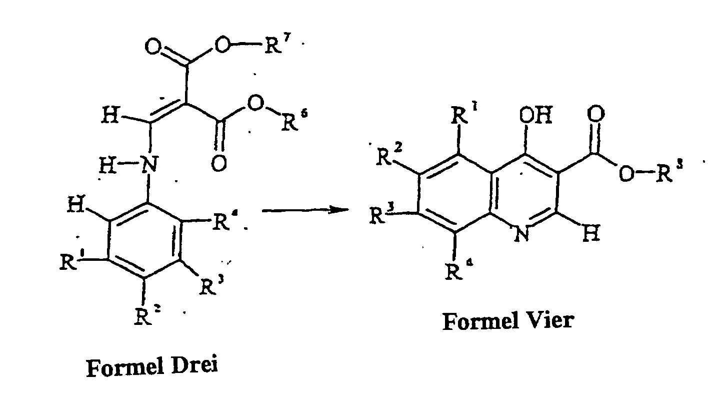 worin R⁸ ein (C₁-C₄)-Alkyl ist;
(3) Umsetzung der Verbindung der Formel vier mit einer Verbindung der Formel fünf, um eine Verbindung der Formel sechs darzustellen, 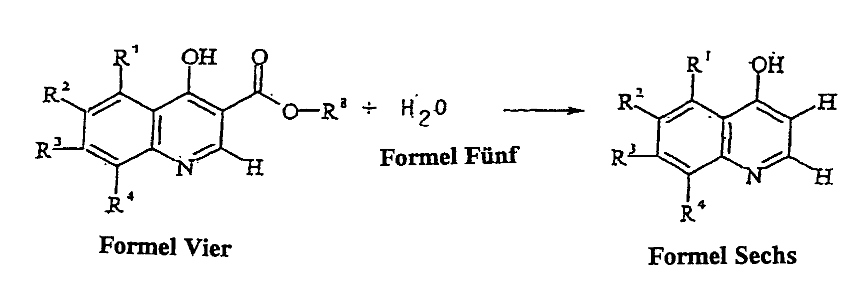 worin H₂O mit der -C(=O)-O-R⁸-Gruppe an Verbindung Vier wechselwirkt, um eine Verbindung der Formel sechs darzustellen;
(4) Umsetzung der Verbindung der Formel sechs mit einer Verbindung der Formel sieben, um eine Verbindung der Formel acht darzustellen, 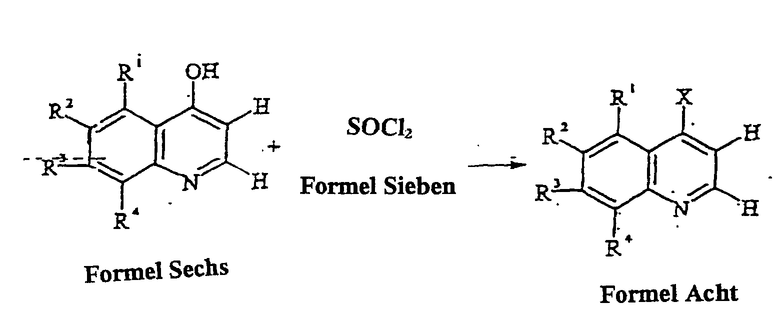 worin SOCl₂ mit der -OH-Gruppe an Verbindung Sechs wechselwirkt, um eine Verbindung der Formel Acht darzustellen; und
(5) Umsetzung der Verbindung der Formel acht mit einer Verbindung der Formel neun, um eine Verbindung der Formel zehn darzustellen 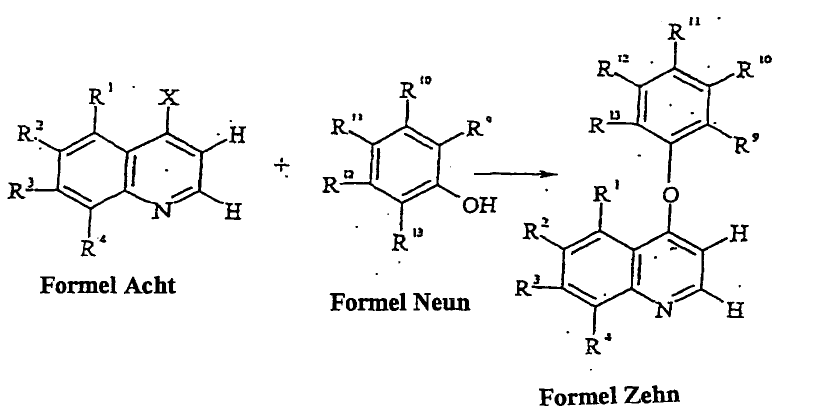 worin R⁹ bis R¹³ unabhängig H, CN, NO₂, OH, Halogen, (C₁-C₄)-Alkyl, (C₂-C₄)-Alkanoyl, Halogen-(C₁-C₇)-Alkyl, Hydroxy-(C₁-C₇)-Alkyl, (C₁-C₇)-Alkoxy, Halogen-(C₁-C₇)-Alkoxy, (C₁-C₇)-Alkylthio, Halogen-(C₁-C₇)-Alkylthio, Phenyl, substituiertes Phenyl, Phenoxy, substituiertes Phenoxy, Phenylthio, substituiertes Phenylthio, Phenyl-(C₁-C₄)-Alkyl, substituiertes Phenyl-(C₁-C₄)-Alkyl, Benzoyl, SiR²⁰R²¹R²² oder OSiR²⁰R²¹R²² sind, wobei R²⁰, R²¹ und R²² Wasserstoff, eine (C₁-C₆)-Alkyl-Gruppe, Phenyl oder substituiertes Phenyl sind, mit der Maßgabe, dass wenigstens einer von R²⁰, R²¹ und R²² anders ist als H, oder R¹¹ und R¹² oder R¹² und R¹³ kombinieren unter Ausgestaltung eines carbocyclischen Rings, und mit der Maßgabe, dass wenn nicht alle von R⁹ bis R¹³ Wasserstoff oder Fluor sind, dann wenigstens zwei von R⁹ bis R¹³ Wasserstoff sind.

2. Ein Verfahren gemäß Anspruch 1, worin wenigstens einer der Schritte (1) - (5) in einem Lösungsmittel ausgeführt wird.

3. Ein Verfahren gemäß Anspruch 2, worin das Lösungsmittel ausgewählt ist aus der Gruppe, die besteht aus Sulfolan, Tetraglykolether, Polyether, Langketten-(C₁₀₋₄₀)-Alkylaromaten, C₁₋₆-alkyliertem Naphtalen, Naphthalen, Diesel, Schweröl und Mischungen hiervon.

4. Ein Verfahren gemäß Anspruch 3, worin die Verbindung der Formel eins 3,5-Dichloranilin ist und die Verbindung der Formel zwei Diethylethoxymethylenmalonat ist.

5. Ein Verfahren gemäß Anspruch 4, worin Dodecylbenzol als Lösungsmittel in den Schritten (1) und (2) verwendet wird und Sulfolan als Lösungsmittel in den Schritten (3) bis (5) verwendet wird.

6. Ein Verfahren gemäß Anspruch 1, worin Schritt (3) bei einer Temperatur von 180 - 270 °C und bei einem Druck von 5 psia bis 50 psia ausgeführt wird.

## Revendications

1. Procédé qui comprend :
(1) la réaction d'un composé de formule un dans laquelle R¹ et R³ représentent chacun indépendamment un atome d'halogène et R² et R⁴ représentent des atomes d'hydrogène, ou bien R³ représente un atome d'halogène, R¹ représente un atome d'halogène ou d'hydrogène, et R² et R⁴ représentent des atomes d'hydrogène, ou bien R⁴ représente un atome d'halogène et R¹ à R³ représentent des atomes d'hydrogène, avec un composé de formule deux dans laquelle R⁵ représente un groupe alkyle en C₁ à C₄ et R⁶ et R⁷ représentent chacun indépendamment un groupe alkyle en C₁ à C₄, pour produire un composé de formule trois, selon le schéma réactionnel suivant : 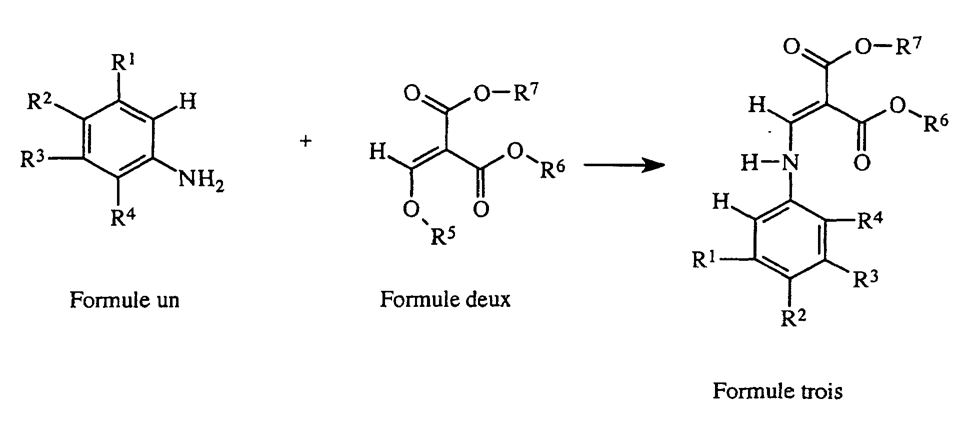
(2) la réaction dudit composé de formule trois pour produire un composé de formule quatre dans laquelle R⁸ représente un groupe alkyle en C₁ à C₄, selon le schéma réactionnel suivant : 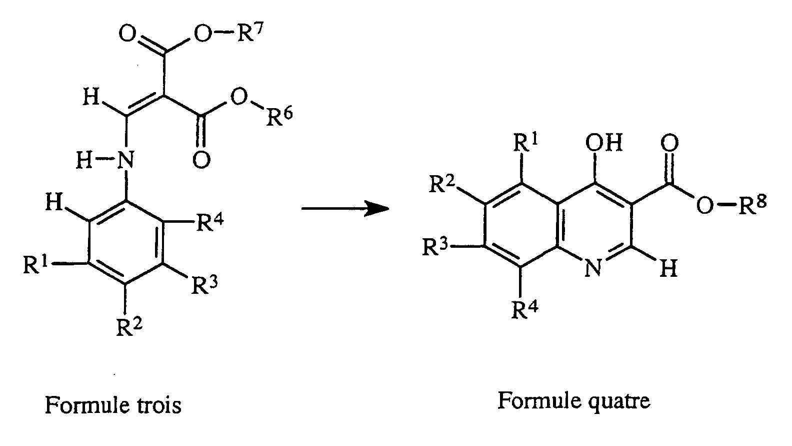
(3) la réaction dudit composé de formule quatre avec un composé de formule cinq pour produire un composé de formule six, selon le schéma réactionnel suivant : 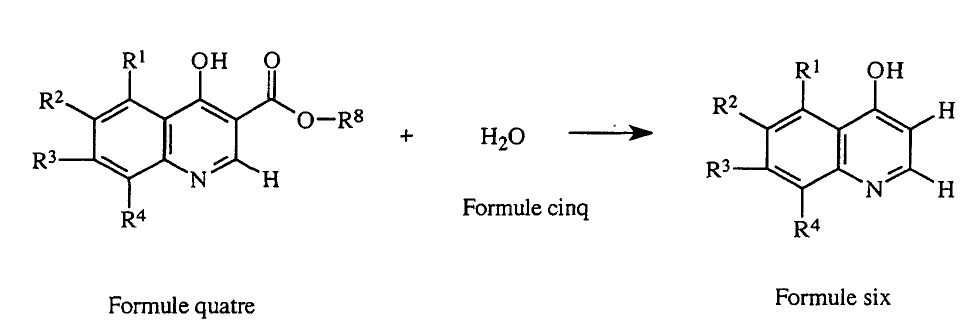 selon lequel l'eau H₂O réagit avec le groupe -C(=O)-O-R⁸ du composé 4 pour produire un composé de formule six,
(4) la réaction dudit composé de formule six avec un composé de formule sept pour produire un composé de formule huit, selon le schéma réactionnel suivant : 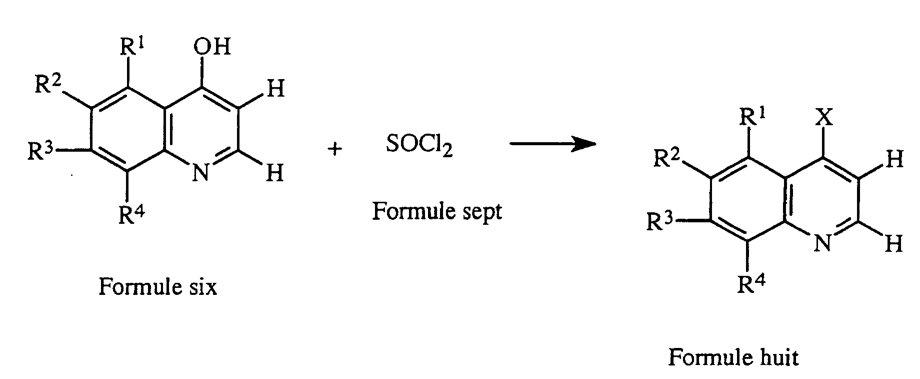 selon lequel SOCl₂ réagit avec le groupe OH du composé 6 pour produire un composé de formule huit, et
(5) la réaction dudit composé de formule huit avec un composé de formule neuf dans laquelle R⁹ à R¹³ représentent chacun indépendamment H, CN, NO₂, OH, un atome d'halogène, un groupe alkyle en C₁ à C₄, un groupe alcanoyle en C₂ à C₄, un groupe halogénoalkyle en C₁ à C₇, un groupe hydroxyalkyle en C₁ à C₇, un groupe alcoxy en C₁ à C₇, un groupe halogénoalcoxy en C₁ à C₇, un groupe alkylthio en C₁ à C₇, un groupe halogénoalkylthio en C₁ à C₇, un groupe phényle, un groupe phényle substitué, un groupe phénoxy, un groupe phénoxy substitué, un groupe phénylthio, un groupe phénylthio substitué, un groupe phénylalkyle(C₁ à C₄), un groupe phénylalkyle(C₁ à C₄) substitué, un groupe benzoyle, un groupe SiR²⁰R²¹R²² ou un groupe OSiR²⁰R²¹R²², R²⁰, R²¹ et R²² représentant chacun un atome d'hydrogène, un groupe alkyle en C₁ à C₆, un groupe phényle ou un groupe phényle substitué, étant entendu qu'au moins l'un de R²⁰, R²¹ et R²² est autre qu'un atome d'hydrogène, ou bien R¹¹ et R¹² ou R¹² et R¹³ sont combinés pour former un noyau carbocyclique, et étant entendu que, à moins que tous les substituants R⁹ à R¹³ soient des atomes d'hydrogène ou de fluor, au moins deux des substituants R⁹ à R¹³ sont des atomes d'hydrogène, pour produire un composé de formule dix, suivant le schéma réactionnel suivant :

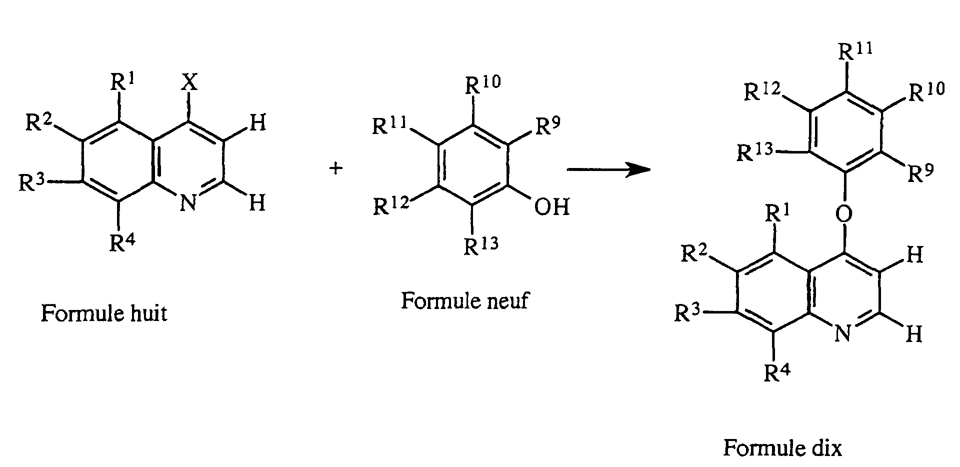

2. Procédé selon la revendication 1, dans lequel au moins l'une des étapes (1) à (5) est réalisée dans un solvant.

3. Procédé selon la revendication 2, dans lequel ledit solvant est un solvant choisi parmi le sulfolane, le tétraglyme, les polyéthers, les composés aromatiques portant un groupe alkyle à longue chaîne (C₁₀ à C₄₀), les alkyl(C₁ à C₆)naphtalènes, le naphtalène, l'huile diesel, le fuel et leurs mélanges.

4. Procédé selon la revendication 3, dans lequel ledit composé de formule un est la 3,5-dichloroaniline et ledit composé de formule deux est l'éthoxyméthylènemalonate de diéthyle.

5. Procédé selon la revendication 4, dans lequel on utilise du dodécylbenzène comme solvant dans les étapes (1) et (2), et on utilise du sulfolane comme solvant dans les étapes (3) à (5).

6. Procédé selon la revendication 1, dans lequel on réalise l'étape (3) à une température de 180 à 270 °C et à une pression de 5 psia à 50 psi a.
